# EUROPEAN PATENT APPLICATION

(11) **EP 2 241 886 A1**
(43) Date of publication of application: **20.10.2010**
(21) Application number: 09005395.0
(22) Date of filing: 15.04.2009
(51) Int. Cl.: G01N 33/543

(54) **Homogeneous agglutination immunoassay method and kit for such method**

(71) Applicant: Beckman Coulter Biomedical Limited, Dublin 2 (IE)
(72) Inventor: Burkhard, Martin, Dr., Ennis, Co. Clare (IE)
(74) Representative: Müller-Boré & Partner Patentanwälte

(57) **Abstract**

A homogeneous agglutination immunoassay method wherein a sample possibly containing the analyte to be measured is mixed with a binding partner for the analyte and with at least one chaotropic agent, monovalent ions in form of at least one salt, or with a combination of at least one chaotropic agent and monovalent ions in form of at least one salt, measuring at least one signal related to interactions of the binding partner with the analyte over a reaction time and calculating from at least one of the signals measured the concentration of analyte in the sample.

## Description

The invention relates to a method for a homogeneous agglutination assay and a kit for such method.

Homogeneous agglutination assays are commonly used to determine the concentration of an analyte in a sample using a binding reaction between specific immunopartners for detection and quantification of the analyte. In such assays the degree of agglutination due to aggregate formation is measured and used as basis for analyte calculation. As a rule in homogeneous assays the sample to be analysed is mixed with a binding partner and after an optional period of incubation the agglutination in the assay is directly measured, without any separation steps necessary.

Usually the binding partner is an antibody specific for the analyte in question. Suited analytes must expose two or more homogeneous or heterogeneous epitopes for the binding partners. Naturally it is also possible to use antigens as binding partner if antibodies are the analytes of interest.

Even if not limited thereto the invention is especially directed to a special type of agglutination assays which is commonly referred to as microparticle enhanced agglutination assay. Such agglutination assays employ microparticles coated with a binding partner for the analyte in question.

The binding partner used is capable of specifically interacting with the analyte in question by e.g. forming antibody-antigen aggregates. The formation of such aggregates (agglutination) in a sample can be monitored by e.g. turbidimetric or nephelometric measurement over a given time. The degree of agglutination measured is an indication on the concentration of analyte in the sample and can be determined by using appropriate calibration curves.

In a typical method the assay is started by adding microparticles coated e.g. with antibodies to a sample/buffer mixture. If the analyte in question is present in the sample, aggregation of microparticles will occur due to the fact that two or more particles can be linked together by binding of at least 2 antibodies on different particles to the same analyte Detection of agglutination can be done by turbidimetric or nephelometric measurement with the wavelength used being chosen depending on the light scattering properties of the microparticles. As a rule the wave length is greater than the mean diameter of the microparticles. On the basis of the agglutination detected in a sample the analyte concentration is calculated by means of a calibration curve.

One main drawback of known microparticle agglutination assays is their limited dynamic range. The term "dynamic range" shall designate the range of concentration in which the assay produces accurate unambiguous results, especially the range in which the dose response curve is linear.

The normal dynamic range of known assays, i.e. the range between detection limit and upper measuring limit, is two orders of magnitude. A lot of analytes, e.g. C-reactive protein (CRP) or Human Choriongonadotropin (HCG) to give only two examples, can have sample concentrations in several orders of magnitude which means that in some cases assays must be repeated or series of dilutions must be performed respectively.

In case of samples containing high concentrations of analyte, one even can observe, that no or only reduced agglutination occurs. If such samples are sufficiently diluted, agglutination corresponding to the concentration of analyte will occur.

The lack or reduction of agglutination at high analyte-concentrations is called prozone effect or "hook effect" and is related to the fact that e.g. excess of an antigen in a sample results in formation of small antibody-antigen aggregates which do not clump to form visible agglutination. In general one can say that the higher the concentration of an antigen is in comparison to that of an antibody, the lower is the signal observed in samples showing the prozone effect.

As increasing concentration of antigen exceeds the relative capability of the population of antibodies to recognise and bind, having the consequence that the degree of agglutination in the sample is not anymore representative of the analyte concentration, resulting in a decrease of signal. This effect is dynamic and represents the change in reaction equilibrium from antigen-antibody aggregate formation to a higher probability of non-aggregated forms predominating.

Known immunoassay methods provide different approaches to broaden the dynamic range of agglutination assays.

US patent 6,248,597 discloses a reagent for agglutination assays which includes two different populations of microparticles. The microparticles of one population have stronger light scattering properties compared to microparticles of the other population and additionally are coated with a binding partner having a higher reactivity towards the analyte compared to the binding partner coated onto microparticles of the other population. According to US 6,248,597 the mix of both microparticle populations provides a broader dynamic range. The particles being coated with antibodies of higher affinity cover the sensitive portion of the measuring range, while the particles coated with antibodies of lower affinity provide detectable agglutination with high analyte concentrations.

A further agglutination assay is described by US-patent 4,590,169. Also this assay works with two different types of antibodies, optionally coated to different types of particles. One of the antibodies has a lower affinity constant for the analyte than the other antibody and is provided in an amount to avoid false negative effects at high antigen concentrations.

The drawback of the known methods is that they require different epitopes on the antigen which can be easily sterically approached. This is not the case with all clinical analytes. Additionally the requirement to use different antibodies in the known assays presents a disadvantage.

The object of the present invention is to provide a method for a homogeneous agglutination immunoassay which compared to known methods has an increased dynamic range allowing measurement of all clinical relevant concentrations of analyte without the risk of false negative results. A further object is to provide a kit which can be used in such agglutination assay.

Said objects are realised by a method according to claim 1 and a kit according to claim 13.

The homogeneous agglutination immunoassay method according to the invention provides that a sample which shall be examined for the concentration of an analyte possibly contained therein is mixed with a binding partner for the analyte and with either at least one chaotropic agent, or with monovalent ions in form of at least one salt or with a combination of at least one chaotropic agent with monovalent ions in the concentrations as defined in claim 1. If not otherwise indicated all concentration values refer to the concentration of the respective component in the assay used for measurement (measuring assay).

To allow a better understanding reference is made to table 1 which refers to the solubility of Urea and NaCI and combinations thereof at 37 C° in a buffer (Tris-Buffer, pH 7,4) commonly used for immunoassay. Urea is a preferred chaotropic agent and NaCl is a preferred salt used in the method according to the invention. Table 1 shows the approximative saturation concentration for Urea, the approximative saturation concentration for NaCl and some pairs of value for different combinations of concentrations of Urea and salt in which saturation occurs.

**Table 1**

| | **Urea mol/l** | **NaCl mol/l** |
|---|---|---|
| **Saturation conc. of NaCl** | 0 | 5 |
| **Saturation conc. of combination** | 2 | 4 |
| **Saturation conc. of combination** | 4 | 3 |
| **Saturation conc. of combination** | 8 | 2 |
| **Saturation conc. of Urea** | 10 | 0 |

From table 1 one can take that saturation of the measuring assay with Urea will occur at a concentration of approximately 10 mol/l, meaning that the claimed lower limit of 40% saturation concentration corresponds to 4 mol/l at the given conditions.

Preferably higher concentrations of chaotropic agent in excess of 50 % of the saturation concentration more preferable in excess of 60% are used.

The same is applicable to the salt concentrations used according to the invention. Saturation of the measuring assay with NaCl will occur at a concentration of approx. 5 mol/l, meaning that the claimed lower limit of 40% saturation concentration corresponds to 2 mol/l at these conditions.

Also here preferably higher salt concentrations in excess of 50 % of the saturation concentration, more preferable in excess of 60% are used.

The third alternative of the invention relates to a use of a combination of chaotropic agent and monovalent ions.

As claimed the concentration of the combination in the measuring assay is in excess of 70 % of the saturation concentration. To take one example saturation will occur when using a combination of 4 mol/l Urea and 3 mol/l. In this case the claimed lower limit of 70 % saturation concentration would correspond to a combination of 2,8 mol/l Urea and 2,1 mol/l NaCl. It is understood that any other pair of values can be calculated in the same manner. Additionally it is no problem for a person skilled in the art to determine the values of a saturation curve for different combinations of chaotropic agent and salt.

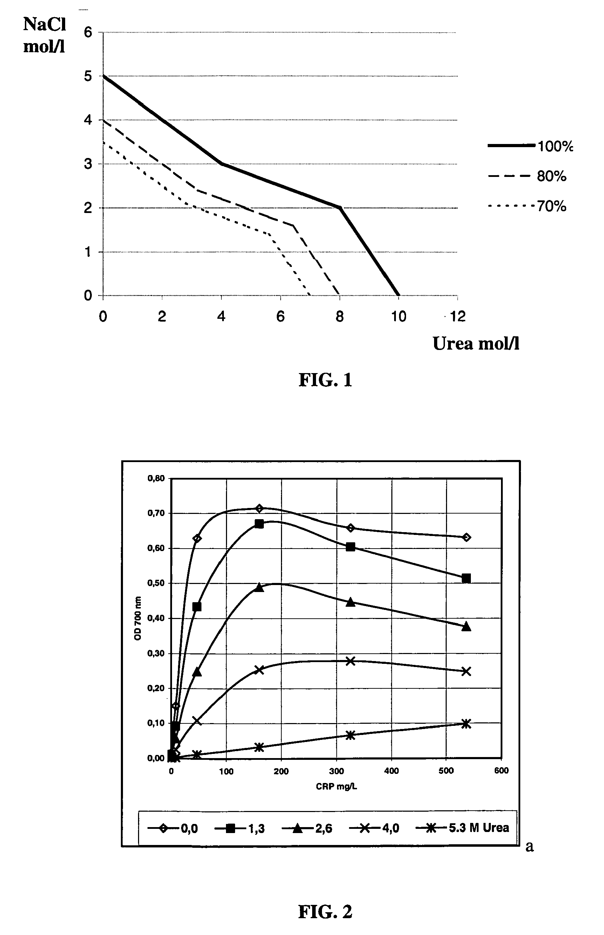

To further exemplify the claimed concentration range of the combination of chaotropic agent and salt reference is made to Fig. 1, in which different Urea concentrations (x-axis) are plotted versus different NaCl-concentrations (y-axis). The continuous line defines the concentration in which saturation occurs, while coordinates lying on the lower dashed line give concentrations of Urea and salt corresponding to 70 % of the saturation concentration and coordinates lying on the upper dashed line correspond to 80 % of the saturation concentration of the combination.

Preferably for the combination concentrations of chaotropic agent and salt in excess of 80% saturation more preferably in excess of 90 % saturation are used. The different alternatives covered by the invention define minimum concentrations of either the chaotropic agent present in the measuring assay or the salt or the combination of both components.

It has turned out that by using one of the components in relative high concentrations the desired broadening of the dynamic range can be obtained. However, it shall be noted that naturally also e.g. in the first alternative defining a minimum concentration of chaotropic agent the measuring assay can also contain a salt in any possible concentration. The same is true for the second alternative which additionally to the claimed salt can include chaotropic agent in a possible concentration. The third alternative relating to a combination of both components covers a special aspect of the invention, namely if it is desired to use both components at the same time in high concentrations.

In the method according to the invention different chaotropic agents can be used. Examples for such agents are Urea, guanidium chloride or hydrochloride, or perchlorates (already mentioned above) to give only some examples. Naturally the invention is not limited to the use of one agent. A mixture of agents can be used as well as stated above.

Typical samples which can be analysed by the method according to the invention are e.g. blood, serum or urine. In general any body liquid or tissue extract shall be encompassed by the term sample which can be examined in an immunoassay. The sample can be used as it is and also in diluted form.

Chaotropic agents are substances which disrupt the three dimensional structure in macromolecules such as proteins, DNA or RNA. Chaotropic agents interfere with stabilizing intra-molecular interactions mediated by non-covalent forces such as hydrogen bonds, van der Waals forces, and hydrophobic effects. They include for example Urea, Guanidium chloride and Lithium perchlorate.

The chaotropic agents used in the invention are not limited to one special agent. It is also possible to use mixtures of 2 or more different chaotropic agents.

In the second alternative of the invention monovalent ions are added to the assay. Preferred monovalent ions are positively charged ions like e.g. NH₄⁺, K⁺, Na⁺, Li+. Addition of these monovalent ions can be done by adding the corresponding salts like (NH₄)₂SO₄, NaCl or LiCl.

Also the use of monovalent ions is not limited to one special ion. It is also possible to use mixtures of 2 or more different monovalent ions.

What was specified above with respect to the single components is also true for the combination of chaotropic agent and salt falling under the third alternative of the invention. Also here the different substances indicated above can be preferably used. It is possible to combine one of more different salts with one or more chaotropic agents. A preferred combination includes Urea and NaCl.

The mixing of the sample with the binding partner, the chaotropic agent and the optional monovalent ions must not necessarily be done in a special sequence. Any possible sequence of mixing can be used. It is only important that the obtained mixture used for measurement (measuring assay) includes all the components.

After mixing, the assay can be optionally incubated over a certain time period and then signals related to interactions of the binding partner with the analyte in the measuring assay are measured over a given reaction time

In a final step the concentration of analyte in the sample is calculated from at least one of the signals measured.

It has surprisingly turned out that as shown by the examples the method according to the invention displays a much broader dynamic range compared to assays known from the prior art allowing the measurement of high analyte concentrations. More surprisingly it has turned out that broadening of the dynamic range does not lead to a decrease of sensitivity at lower analyte concentrations.

The theory is that by adding chaotropic agent to the assay the affinity of the antibody-antigen reaction is decreased with the consequence that at high analyte concentrations the reaction equilibrium between antigen-antibody aggregates and non-aggregated forms is shifted towards the aggregates. More generally spoken one can say that the addition of chaotropic agents allows a modulation (in the sense of selective reduction) of e.g. the affinity of the antibodies used to a special desired affinity-value depending on the concentration of chaotropic agent used.

The possible modulation of the affinity in the method according to the invention is especially advantageous when e.g. polyclonal antibodies are used. In some cases polyclonal antibodies can show lot to lot variations in their affinity. These variations can be easily balanced by the selective addition of chaotropic agent to the assays.

A further advantage is that the addition of chaotropic agents in the mentioned concentration changes the refraction index of the solution in the measuring assay compared to an assay buffer containing no or little chaotropic agents. As a consequence the blank value of such assays decreases which means that the signal to noise ratio increases. Finally it has turned out that when using chaotropic agent in an assay, the stabilisers and preservatives required in prior art assays can be omitted.

Addition of monovalent ions on the other side slows down the movement of the binding partners, especially of particles coated with the binding partners in the assay with the consequence that their collision frequency decreases. Also this effect contributes to aggregate formation and helps to avoid the hook effect at high analyte concentrations. In case of lower analyte concentrations the method of the invention compared to assays without chaotropic agent and monovalent ions will have a somehow slower kinetic which, however, can be easily compensated by slightly extending the reaction in time in the measuring step.

Up to now homogeneous assays working with a high concentration of chaotropic agent, especially in combination with a high concentration of monovalent ions, as in the third alternative of the invention, are not known in the prior art.

There are some assays known, which use chaotropic agents. In this context reference is made to EP 0 038 181 and EP 0 638 806. In the assays described in both references Urea is added. However, both assays described in the cited documents are heterogeneous assays and the purpose of the Urea-addition is to avoid non-specific interactions or to prevent assay-drift respectively. The use of chaotropic agents in homogneous assays in order to seletively reduce e.g. affinity of the antigen-antibody reaction is not mentioned or suggested in these documents.

Different embodiments of the method according to the invention are addressed to in further subclaims.

The method of the invention can be effected manually or more preferably in automatic analysers. Especially if automatic analysers are used mixing of the sample is preferably performed in a standardised 3 step-manner as following:

To begin with a first reagent A is provided. Then the sample is added to reagent A and a further reagent B is added to yield the measuring assay.

Preferably reagent B contains the binding partner, preferably coated to microparticles, and either reagent A or reagent B or both contain the chaotropic agent and/or the optional monovalent ions

A preferred method provides that the chaotropic agent and monovalent ions are contained in reagent A, while reagent B includes monovalent ions besides the binding partner, preferably coated to particles. In this case most preferably the concentration of monovalent ions in reagent A corresponds to the concentration in reagent B.

The assay is preferably performed at temperatures between 4°C and 45°C with an optimum value at 37°C.

The immunoassay method of the invention works with all known agglutination or haem-agglutination tests with and without particle enhancement.

Preferred are particle enhanced assays. A further embodiment of the invention therefore provides that the binding partner is immobilized on micro particles, especially e.g. on polymeric beads like e.g. latex beads. However, any particle is suited which can be used in common immunoassays or micro particle enhanced light scattering agglutination assays, respectively. The advantage compared to direct agglutination is that by using immunoreactive substance coupled to micro particles, the sensitivity of the assay is increased.

The diameter of the particles chosen is preferably between 50 mn and 500 nm. Depending on the settings of the assay different particle diameters can be used with good results. As a general rule one can say that smaller particles in a range from 50 to 100 nm create less hook effect, i.e. the dynamic range increases, but also show a lower sensitivity. In contrast thereto larger particles with a diameter in a range from 100 to 500 nm produce an earlier hook-effect but show a higher sensitivity due to their higher absorption values as will be discussed later in connection with the example.

In view of the above a preferred embodiment of the invention provides that a mixture of at least 2 populations of particles of different diameter is used, preferably one of the populations including particles with a diameter between 50 nm and 100 nm and the other population including particles with a diameter between 100 nm and 500 nm, with the mix ratio of the at least 2 populations lying between 1:99 and 99:1, preferably between 20:80 and 80:20, most preferably between 40:60 and 60:40.

Naturally also mixtures with particle populations of further diameters falling within the range of 50 to 500 nm can be used.

Preferably the method of the invention uses turbidimetric techniques for determining interaction between e.g. antibodies and antigen. However, also other techniques which allow quantification of said interactions and which suffer from the prozone effect, can be used, like e.g. chemiluminescence, enzyme immunoassays, fluorescence or nephelometry to give only some examples. For that reason, even if herein it is mainly referred to turbidimetric measurement, the invention shall not be limited to the use of this technique.

Turbidimetric assays can be performed by using a spectrophotometer or turbidimeter. The used wavelength depends on the assay and depends mainly on the diameter of the particles. In general agglutination in particle enhanced assays can preferably be measured in a range between 450 - 800 nm, especially preferred in a range between 500 and 700.

As stated above the method according to the invention is using an antibody-antigen reaction in which both antigen or antibody can be the analytes while the remaining immunoreactive component is the binding partner preferably coated on the particles.

In most cases the analyte to be measured is an antigen. Typical examples for such analytes are cancer markers like e.g. prostatic specific antigen (PSA) or HCG or inflammatory markers like CRP to give only some examples of markers which can be especially affected by the prozone phenomenon.. Naturally all further analytes which interact with an antibody can be detected using the method according to the invention.

The antibodies used in the method according to the invention must be capable to bind to the analytes in question. The term antibody encompasses monoclonal and polyclonal antibodies. Additionally also fragments, like Fab, (Fab₂)-fragments, can be used.

Preferably the method of the invention uses polyclonal antibodies. The advantage in using polyclonal antibodies is that they bind to different heterogeneous epitopes on the analytes while monoclonal antibodies as a rule need repetitive homogeneous epitopes on the analyte which are not always available. Additionally polyclonal antibodies are less expensive compared to monoclonal antibodies. Their main draw back which is that their affinity can vary from lot to lot However, this draw back can be easily compensated by choosing a suitable concentration of chaotropic agent in the method.

Preferably the antibodies used in the method to the invention have a high affinity or avidity to the analyte. High affinity or avidity shall mean that the dissociation constant of the antibody-antigen reaction is generally equal or lower than 10⁻⁷ mol/l at the temperature used for measurement. Preferably the dissociation constant is lower or equal than 10⁻⁸ mol/l, most preferably lower or equal than 10⁻⁹ mol/l. The use of high-affinity antibodies allows a modulation of their affinity in a very broad range.

A further important advantage is that compared to prior art assays which need 2 different types of antibodies the method according to the invention only requires one antibody to cover a broader dynamic range.

The invention is not limited to antibody-antigen reaction. Analyte and binding partner can also be combinations of receptor and ligands, avidine or streptavidine and biotine, protein A and immunoglobuline, cellreceptors and agonists or antagonists.

Furthermore the invention is also directed to a kit which can be used in performing the assay. The kit includes the reagents A and B mentioned above.

In the following the invention shall be illustrated by means of a 2 examples and accompanying Figs. 2-5 showing the assay performance at different settings.

### EXAMPLE 1

### 1) Reagents

The reagents used had the following composition:

**i) Reagent A**

| Component | Molarity |
|---|---|
| Tris HCl buffer pH 7.4 | 200- 300 mmol/L |
| Urea | 5 different concentrations were used, namely: 0 mol/l, 2,0 mol/l, 4,0 mol/l, 6,0 mol/l, and 8,0 mol/l. |

H₂O was added up to 1000ml. Optionally preservatives, stabilisers, EDTA or proteins can be added to reagent A. However, they are not necessary for the method according to the invention.

**ii) Reagent B**

| Component | Molarity | |
|---|---|---|
| MQPSO pH 7.4 | 18 m mol/l | |
| NaCl | 3.6 mol/l | |
| CRP-antibody coated latex polystyrene microparticles | 0,2% solid | 2 different particle sizes were used, namely: 101 nm and 158 nm |

H₂O was added up to 1000ml. Optionally preservatives, stabilisers, EDTA, proteins, or detergents can be included in reagent B. However, they are not necessary for the method according to the invention.

### iii) Calibrators

A set of calibrators in a concentration range of 0-530 mg/l CRP.

### 2) Assay

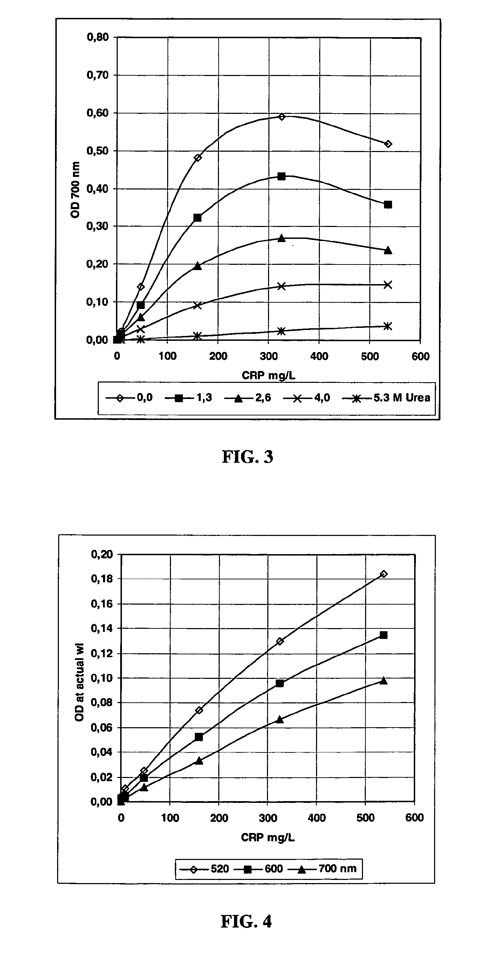

The assay was performed in an automatic analyser Olympus AU2700 at 37C°. Reagent A was used with all different Urea concentrations in combination with each of the particle sizes of reagent B.
- 2 µL of calibrator were pipetted into 150 µL of reagent A and mixed
- 3.2 minutes later 75 µL of reagent B was added and mixed.
- Then the OD at 700 nm was measured over 4.9 minutes.
- The change in OD was expressed as the change between beginning and end of the measuring period.
- The changes in OD were plotted against the concentration of the CRP in the individual calibrators (Figures 2 and 3).

The dose response curve with the highest hook effect concentration was identified and the Urea concentration of this dose response curve used for Example 2.

### EXAMPLE 2

### 1) Reagents

Reagent A with a Urea concentration of 8,0 mol/l was selected. As in Example 1 reagent B could include either particles of 158 nm diameter of 101 nm diameter.

### 2) Assay

Similar to example 1 the whole assay was performed at 37°C in an Olympus AU2700 analyzer.
- 2 µL of each calibrator were pipetted into 150 µL of reagent A and mixed
- 3.2 minutes later 75 µL of the reagent B was added and mixed.
- Then the OD at 520nm, 600 nm or 700 nm was measured over 4.9 minutes.
- For each of the wavelengths the changes in OD was expressed as the change between beginning and end of the measuring period.
- For each of the wavelenghts the changes in OD were plotted against the concentration of the CRP in the individual calibrators (Figures 4 (158 nm) and 5 (101 nm)).

Figs. 2 and 3 show the results of assays of Example 1 which use 2 different particle diameters at different Urea concentrations.

The particles used to generate the curves shown in Fig. 2 had a diameter of 158 nm and the Urea concentrations resulting in the measuring assay were 0; 1,3; 2,6; 4,0 and 5,3 mol/l. The measurement was done at a wavelength of 700 nm. Under these conditions all curves made from the values measured at the different Urea concentrations with the exception of the curve for 5,3 mol/l Urea concentration showed a hook effect.

The particles used to generate the curves shown in Fig. 3 had a diameter of 101 nm. Apart therefrom the setting were the same as in Fig.1. Also here most curves showed a hook effect, with exception of the curves generated for 4,0 and 5,3 mol/l Urea.

Both Figs. 2 and 3 show a dependence of the dynamic range on the concentration of Urea. When comparing Figs 1 and 2 it appears that the settings of Fig. 2 lead to a little broader dynamic range compared to Fig. 1. On the other hand the OD values measured in the assays shown in Fig. 2 are higher, which means that the assays have a higher sensitivity compared to the assays shown in Fig. 3.

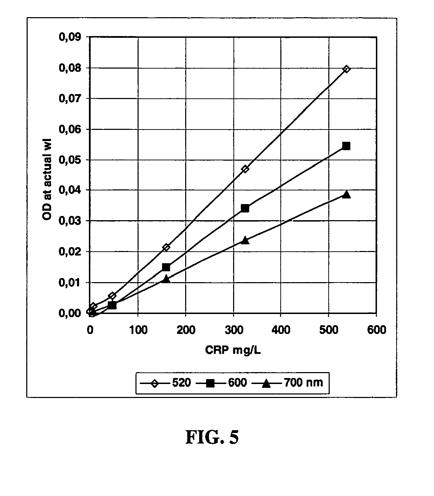

Figs. 4 and 5 show the results of assays performed according to Example 2. Here again assays were performed which used particles of the different diameters 158 nm and 101 nm. Only one Urea concentration, namely 5,3 mol/l in the measuring assay (resulting from the addition of reagent A with 8 mol/l Urea), was used. Measurement of the assays was done at 520, 600 and 700 nm.

Under these conditions none of the curves generated a hook effect. The assays using the smaller particles produce more linear curves while again the assays with the bigger particles have a higher sensitivity.

All 4 Figures show that by adjusting the different settings the method of the invention can be optimised in a very easy manner.

Additionally further assays were done using combinations of Urea and NaCl falling under the third alternative of the invention. Also here a broader dynamic range could be obtained. When using e.g. concentrations of 4 mol/l and 2,8 mol/l Urea in the measuring assay, a dose response curve (not shown) could be obtained with a dynamic range up to 600 mg/l CRP.

## Claims

1. A homogeneous agglutination immunoassay method wherein:
(a) a sample possibly containing the analyte to be measured is mixed with
- a binding partner for the analyte and with
- at least one chaotropic agent, with the concentration of chaotropic agent adjusted such that the final concentration of chaotropic agent in the assay used for measurement in step (b) (measuring assay) is in excess of 40% of its saturation concentration in the measuring assay at the temperature used for the measurement, or with
- monovalent ions in form of at least one salt, with the concentration of salt adjusted such that the final concentration of salt in the measuring assay is in excess of 40% of its saturation concentration at the temperature used for the measurement, or with
- a combination of at least one chaotropic agent and monovalent ions in form of at least one salt,
with the concentration of the chaotropic agent and the salt adjusted such that the final concentration of the combination is in excess of 70% of the saturation concentration at the temperature used for the measurement
(b) measuring at least one signal related to interactions of the binding partner with the analyte in the measuring assay over a reaction time and
(c) calculating from at least one of the signals measured in step (b) the concentration of analyte in the sample.

2. Method according to claim 1, **characterised in that** the assay is a particle enhanced assay with the binding partner being immobilised on microparticles.

3. Method according to claim 2, **characterised in that** the diameter of the particles chosen is preferably between 50 nm and 500 nm.

4. Method according to claims 2 or 3, **characterised in that** a mixture of at least 2 populations of particles of different diameter is used.

5. Method according to claim 4, **characterised in that** one of the populations includes particles with a diameter between 50 nm and 100 nm and the other population includes particles with a diameter between 100 nm and 500 nm, with the mix ratio of the at least 2 populations lying between 1:99 and 99:1, preferably between 20:80 and 80:20 and most preferably between 40:60 and 60:40.

6. Method according to claims 1-5, **characterised in that** the chaotropic agent is Urea.

7. Method according to claims 1-6, **characterised in that** interaction between binding partner and analyte is determined by turbidimetric techniques.

8. Method according to claim 7, **characterised in that** wavelength used for turbidimetry lies in a range between 450 nm - 800 nm, preferably in a range between 500 nm - 700 nm.

9. Method according to claims 1-8, **characterised in that** the binding partner is an antibody, preferably a polyclonal antibody.

10. Method according to claim 9, **characterised in that** the antibody has a highs affinity or avidity to the analyte.

11. Method according to claims 1-10 wherein the mixing of the sample in step (a) comprises the following:
(a1) providing a first reagent A
(a2) adding of a sample to reagent A, and
(a3) adding of a second reagent B containing particles coated with the binding partner to yield the measuring assay,
wherein either reagent A or reagent B or both contain the chaotropic agent and wherein either reagent A or reagent B or both contain the monovalent ions

12. Method according to claim 11, **characterised in that** the chaotropic agent and monovalent ions are contained in reagent A, while reagent B includes the monovalent ions.

13. Kit for a method according to claim 11, including reagent A and reagent B.
